# EUROPEAN PATENT SPECIFICATION

(11) **EP 2 327 433 B1**
(45) Date of publication and mention of the grant of the patent: **18.04.2012**
(21) Application number: 09177194.9
(22) Date of filing: 26.11.2009
(51) Int. Cl.: A61M 5/158

(54) **Externally triggerable cannula assembly**
Extern auslösbare Kanülenanordnung
Ensemble de canule pouvant être déclenchée de manière externe

(43) Date of publication of application: 01.06.2011
(73) Proprietor: F. Hoffmann-La Roche AG, 4070 Basel (CH); Roche Diagnostics GmbH, 68305 Mannheim (DE)
(72) Inventor: Kaufmann, Heiner, 3008, Bern (CH); Teutsch, David, 3251, Wengi (CH); Scheurer, Simon, 3006 Bern (CH)
(74) Representative: Schwabe - Sandmair - Marx

(56) References cited:
- EP-A1- 1 764 125
- EP-A1- 1 970 084
- WO-A2-02/081012
- US-A1- 2008 319 414
- US-B2- 7 455 663

## Description

The present invention relates to: an externally triggerable cannula assembly for administering a fluid or liquid, in particular a medical product such as liquid insulin; a trigger device for an externally triggerable cannula assembly; a cannula system comprising an externally triggerable cannula assembly and a trigger device; a medical device or diabetes therapy device comprising a trigger device; and a method for delivering a fluid or liquid to a cannula.

A fluid product is often continuously delivered to a body using a cannula which is inserted into the body, wherein the cannula remains in place for several days. Diabetes patients may use such a cannula, which is subcutaneously positioned for a continuous delivery of insulin by a drug infusion pump or for monitoring glucose levels using a sensor. Frequently, the patient initially applies a subcutaneous insertion device which comprises a cannula device for subsequently delivering insulin by means of the pump. Older and/or weakened patients in particular often encounter difficulties in correctly administering the drug due to impaired manual dexterity or lack of motor skills needed to properly handle the cannula device, whence the risk arises that an incorrect dose may be administered or that the cannula device may be unintentionally and/or inappropriately triggered.

In many cases, the insertion device is a separate, reusable device. This approach, however, requires the patient to use an additional device, which is often perceived as being complicated to handle. Therefore, disposable insertion devices are also available, in which the infusion cannula is supplied with and already attached to the insertion device, and the insertion device can be removed from the cannula and disposed of, once insertion is complete.

This approach, however, is rather expensive, since it necessitates a new disposable insertion device every few days, as well as an increased volume of sterile packing for each infusion set, which is known to be critical with respect to user acceptance.

US 2007/0142776 A9 discloses an insertion device and insertion set. The insertion device for inserting at least a portion of at least one piercing member of an insertion set through the skin of a patient includes a device housing, a carrier body and a driver. The carrier body is slidably received within the device housing for movement between an advanced position and a retracted position. The driver is operatively coupled between the device housing and the carrier body to urge the carrier body from the retracted position toward the advanced position to place at least a portion of the at least one piercing member of the insertion set thorough the skin of the patient to install the insertion set to the patient. The receiving structure of the carrier body is removable from the insertion set while maintaining the installation of the insertion set to the patient.

US 2008/0319414 A1 discloses an insertion apparatus and a method for use with a device for delivery of a therapeutic fluid into a body of a patient and/or for sensing of a bodily analyte. The apparatus includes a housing adapted for loading therein at least one cannula cartridge unit having a protective member. The protective member accommodates at least one penetrating cartridge having a subcutaneously insertable element and a penetrating member. The apparatus includes a displacement mechanism capable of protracting the penetrating cartridge towards the body of the patient, where protraction of the penetrating cartridge results in insertion of the subcutaneously insertable element into the body of the patient.

US 2009/0198191 discloses patches for medical devices, wherein in various embodiments, an adhesive patch of a medical device may have selective areas with adhesive material of varying adhesion strengths. In other embodiments, an adhesive patch of a medical device may include adhesive material that may be activated by a catalyst to increase or decrease the adhesion strength of the adhesive material. In further embodiments, a medical device may include a pierceable membrane containing an agent, the pierceable membrane being positioned to be pierced by a needle and to cause some of the agent to be carried to the user-patient.

US 7,455,663 B2 discloses an infusion medium delivery system, a device and method for delivering an infusion medium to a patient-user, including a needle inserter device, and a method for inserting a needle and/or cannula into a patient-user to convey the infusion medium to the patient-user.

WO 2009/004026 A1 discloses an inserter for an infusion set for intermittent or continuous administration of a therapeutic substance, such as for example insulin. The inserter comprises means for insertion and retraction of an introducer needle. With the inserter of this document, it is possible to introduce an insertion needle when placing a medical device sub- or transcutaneously.

EP 1 764 125 A1 discloses a system comprising an insertion head and an inserter. The insertion head comprises a cannula to be penetrated into the skin of a patient. The inserter comprises a spring which projects the tip of a cannula out of a housing of the inserter if a button is pressed such that the blocking of a hook is released.

WO 02/081012 A2 discloses a cannula application device in which a cannula is applied using a needle. The needle is attached to a needle carrier which is biased using a spring. The movement of the needle carrier is blocked by a lever which can be released by pushing a button.

It is an object of the invention to provide a user-friendly cannula assembly which provides increased security and prevents the cannula assembly from being inadvertently activated.

This object is solved by the devices as defined in the independent claim. Specific embodiments are defined in the dependent claims.

In accordance with one aspect, the invention relates to a cannula assembly for transcutaneous fluid or liquid transport, to be used with a trigger device for triggering an activation mechanism of the cannula assembly. The cannula assembly comprises a cannula module and an inserter module which is in particular disposable.

The cannula module comprises a skin-contacting surface such as an adhesive layer or an attachment to be placed or stably fixed to an outer surface of an object or the skin of a body, in particular the skin of a user. The cannula module also comprises a cannula which is composed of a hollow structural element adapted to protrude forwards through the skin of the user and remain in the tissue for the duration of use, and through which a fluid or drug can be administered. The structural element can be a hollow steel cannula with a pointed tip or a soft and flexible tube, in particular made of Teflon, which is then usually inserted by a guiding needle. The cannula comprises a fluid inlet at a distal end relative to the skin-contacting surface, and a fluid outlet at a proximal end relative to the skin-contacting surface which is adapted to be inserted through the outer surface of the object or the skin of the user, wherein the fluid inlet is in fluid communication with the proximal end portion and with the fluid outlet. In the following description, the cannula is assumed to comprise or be the hollow steel cannula.

The cannula is configured to assume two states: a pre-operational or initial state and an operational or final state. In the pre-operational state, the cannula is retracted relative to the skin-contacting surface such that it does not project from the skin-contacting surface. In the operational state, the cannula is advanced relative to the skin-contacting surface and projects from the skin-contacting surface. In accordance with one embodiment, the needle is adapted to pierce the user's skin, thus enabling a fluid or liquid to be transported from the fluid inlet into the body of the user.

The disposable inserter module is designed to be coupled to the cannula module in the pre-operational state of the cannula. In the operational or piercing state, the cannula can be adapted to penetrate the skin and/or tissue of the body, thus allowing a fluid or liquid to be transported into the body.

In accordance with one embodiment, the inserter module is designed to be releasably coupled to the cannula module and to be separated from the cannula module in the operational state of the cannula.

The inserter module comprises an energy store and the activation mechanism.

The energy store is designed as an energised or energisable actuating means such as a biased spring member which is adapted to transform stored or potential energy into kinetic energy when discharged, or a pyrotechnic material which is adapted to undergo an exothermic chemical reaction in order to produce kinetic energy when ignited, or a gas which is adapted to expand when heated, or an electromagnetic device which is adapted to move when triggered and/or when an electric current is passed through it, or a magnetic device which is adapted to move when triggered.

If the energy store acts as an energised actuating means, it can be prevented from being discharged by blocking means, wherein releasing the blocking means can enable the energy store to be discharged and the cannula to be moved. If the energy store acts as an energisable actuating means, it can be adapted such that it can be discharged by an activating means, wherein activating or firing or triggering the activating means can enable the energy store to be discharged.

The inserter module is configured to move the cannula from the pre-operational state to the operational state using the energy of the energy store or by at least partially discharging the energy store. In accordance with one embodiment, the inserter module is designed to bear the cannula module and to position the cannula at a desired location on the surface of the object, optionally for example at a desired angle to the skin.

The activation mechanism comprising the blocking or activating means is adapted to prevent the energy store from being discharged, thus maintaining the pre-operational state of the cannula, and to enable the energy store to be discharged, thus forcing the cannula into the operational state. "Forcing the cannula" is understood to mean moving or shifting or advancing or pushing or pressing the cannula axially along a casing or body of the inserter module.

The activation mechanism can be triggered from outside the cannula assembly by the trigger device. Triggering can for example involve transferring a force or movement or mechanical impulse from the trigger device to the activation mechanism in order to release the blocking means or initiate the activating means in order to advance the cannula. In accordance with one embodiment, the activation mechanism is designed such that it can only be triggered by a trigger device which matches the activation mechanism, thus fulfilling a dedicated match criterion.

Triggering is possible from outside the cannula assembly. In accordance with one embodiment, triggering is only possible from outside the cannula assembly, wherein it is possible to assemble the trigger device together with a medical device or diabetes therapy device such as a glucose meter or a fluid supply or an infusion pump, wherein the trigger device and the medical or diabetes therapy device thus become a single physical entity. Employing a reusable triggering medical or diabetes therapy device can be beneficial and cost-efficient to the user, since the complexity of the cannula assembly, in particular its disposable modules, can be considerably reduced by relocating the trigger device outside the cannula assembly.

The energy store can in particular be a coil spring or a gas spring, for example a compression or extension spring, which is supported on one side by being axially fixed to the body and on the other side by being axially fixed to a plunger.

The energy store can in particular be a pyrotechnic device which is adapted to ignite a pyrotechnic material or to expand a gas when exogenously triggered, for example by an ignition device, in order to axially advance the plunger and/or the cannula.

In one embodiment, the activation mechanism can comprise a trigger interface which is adapted to receive an exogenous or external trigger signal or trigger effect and to convert the trigger signal or trigger effect into a mechanical and/or magnetic and/or electric and/or electromagnetic and/or hydraulic and/or thermal and/or pneumatic and/or pyrotechnic activating effect. A trigger effect can be a trigger signal, such as a force, a voltage or electric field, or a magnetic field. The activating effect can be a force or momentum which is exerted on a blocking means or blocking element. The blocking element can be designed to prevent the energy store from being discharged, until the blocking element is released from a blocking position in order to discharge the energy store and move the cannula.

A mechanical activating effect can be a force which is exerted, for example by a moving or shifting actuator, on a pivoting retaining hook. Pivoting the retaining hook can release the retaining hook from an engagement with a retaining element, thus enabling the energy store - for example, a biased coil spring or gas spring coupled to the retaining hook - to be discharged.

A magnetic activating effect can be a magnetic force which is exerted, by a permanent magnet or an electromagnet, on a blocking element such as a pivoting retaining hook, the top of which comprises a permanent magnet or an electromagnet.

A pneumatic activating effect can be a pneumatic force exerted for example by releasing a gas spring which is integrated in the trigger device and fluidly connected to the activating mechanism, thus exerting a force or momentum on the activating mechanism.

A thermal activating effect can be achieved by heating a gas contained in the cannula assembly, thus generating a mechanical force or momentum.

A hydraulic activating effect can be achieved by a fluid or liquid such as insulin which is supplied from outside the cannula assembly. In accordance with one embodiment, the liquid can be supplied from the trigger device or from a medical device or diabetes therapy device which is coupled to or integrated with the trigger device, for example in order to wet a spongy element of the activating mechanism, thus changing the consistency of the spongy element and consequently moving a blocking element supported by the spongy element.

An electric activating effect can be an electric field which acts on an activating element, such as an ignition device, in order to enable the energy store, such as a pyrotechnic device, to be discharged. The activating means can be designed to enable the energy store to be discharged when triggered or activated or fired or released.

In one embodiment, the activation mechanism can comprise a security mechanism designed to help prevent the energy store from being accidentally discharged, without having been triggered, as could for example be caused by inadvertent mechanical shocks acting on the cannula assembly and caused by a user before triggering.

The security mechanism can be adapted to switch from a blocking state in which the security mechanism prevents the energy store from being discharged, for example when the trigger device is detached from the cannula assembly, to a releasing state in which the energy store can be discharged. The security mechanism can be adapted to interact with key elements of the trigger device, wherein it is only possible to switch from the blocking state to the releasing state if the key elements of the trigger device and the security mechanism of the activation mechanism fulfil corresponding criteria.

A matching criterion can for example be that a shape of the key elements of the trigger device must fit or match correspondingly shaped elements of the security mechanism in order to allow the security mechanism to enter the releasing state. The security mechanism can for example comprise a retaining pin which is levered on a fulcrum from the blocking state, in which the retaining pin prevents the retaining hook from pivoting, to the releasing state in which the retaining pin is released in order to be pivoted. In accordance with one embodiment, an opening in the trigger device or in a medical or diabetes therapy device comprising the trigger device can be designed to receive a bar-shaped inserter module, wherein the wall enclosing the opening can comprise a key element such as a release buckle which is adapted to release a levered retaining pin of the security mechanism which is adapted to engage with the release buckle when the inserter module is inserted into the opening of the trigger device or medical or diabetes therapy device.

In some embodiments, in particular those involving an electric trigger used for example in a pyrotechnic device, a reed switch which is configured to be closed by a magnet in the triggering device can be provided in the cannula assembly. Alternatively, it is possible to provide an RF transmitter in the triggering device and a corresponding receiver in the cannula assembly. In this case, the matching criterion can be a code which is transmitted from the triggering device.

In a security mechanism such as that described above by way of example, triggering is more specifically only possible when the inserter module is inserted into the opening of the trigger device and the release buckle of the trigger device levers the retaining pin of the inserter module when the inserter module is advanced into the opening of the trigger device or medical or diabetes therapy device.

If triggering is dependent on an external trigger device and an activation mechanism fulfilling certain matching criteria, this can substantially improve the operating security of the cannula assembly by reducing the risk of the cannula assembly being inadvertently or accidentally activated.

In one embodiment, the activation mechanism can comprise or can be designed to operate as a blocking mechanism which prevents the energy store from being discharged, in particular if a mechanical or magnetic or electromagnetic or hydraulic or thermal or pneumatic activating effect is used. The blocking mechanism can be triggered from outside the cannula assembly to be released, thus enabling the energy store to be discharged or resulting in the energy store being discharged.

In one embodiment, the activation mechanism can be adapted to be triggered by moving a blocking element. In accordance with one embodiment, the blocking element forms part of the trigger interface and the blocking mechanism and can thus simultaneously receive an activating effect and control the discharge of the energy store. The blocking element can be releasably attached to a retaining element in order to prevent the energy store from being discharged, and can be adapted to be detached from the retaining element by the activating effect in order to discharge the energy store.

Accordingly, the following security features can be incorporated in order to make handling the cannula assembly more secure:
- the activation mechanism comprises a blocking element and a retaining element;
- the blocking element is releasably attached to the retaining element in order to prevent the energy store from being discharged;
- the blocking element is adapted to be detached from the retaining element by the activating effect;
- detaching the blocking element from the retaining element causes the energy store to be discharged;
- the activation mechanism can be triggered by moving the blocking element;
- the blocking element optionally forms part of the trigger interface.

The inserter module can in particular comprise an elongated cylindrical body, and the cannula module can comprise a plunger mounted in it for a longitudinal shifting movement within the body of the inserter module between a retracted position and an advanced position, wherein the energy store engages with the plunger in order to advance the plunger when the energy store is discharged. The cannula can be axially fixed to the plunger such that advancing the plunger from the retracted position to the advanced position moves the cannula from the non-operational to the operational state.

The retaining elements of the activation mechanism can in particular comprise a retaining hook and a retaining bearing or bearing element which are designed to assume a blocking state in which the retaining hook is releasably engaged with the retaining bearing, and a releasing state in which the retaining hook is released from the retaining bearing, wherein the energy store is prevented from being discharged in the blocking state and allowed to be discharged in the releasing state.

The retaining hook can in particular be a pivoting retaining hook comprising a hooked end which is releasably engaged with the retaining bearing, and an opposite, straight end which pivots on and is axially fixed to the plunger, wherein the pivoting retaining hook can be released from the retaining bearing by a lateral force which pushes against the hooked end of the retaining hook and moves the retaining hook from the blocking state to the releasing state. The retaining hook can in particular be an axially shiftable retaining hook comprising a hooked end which is releasably engaged with the plunger, and an opposite end which is connected, axially fixed and such that it can be separated, to a bistable elastic element having two equilibrium states, wherein the transition of the bistable elastic element from the blocking state to the releasing state enables the retaining hook to transition from the blocking state to the releasing state.

The transition of the bistable elastic element, which is supported by a spongy element having a higher consistency in a dry state and a lower consistency in a moist state, from the blocking state to the releasing state can be configured such that it is enabled by feeding a triggering fluid or liquid to the spongy element.

The retaining hook can in particular be a laterally shiftable retaining hook comprising a first hooked end which is releasably engaged with the plunger, and a second hooked end which is connected, laterally fixed and such that it can be separated, to the bistable elastic element, wherein the bistable elastic element prevents the retaining hook from laterally shifting in the blocking state and enables the retaining hook to laterally shift in the releasing state.

The retaining hook can in particular be a levered retaining hook comprising a hooked end which is releasably engaged with the plunger, and an opposite end which can be moved by an exogenously applied lateral force in order to lever the hook on a fulcrum and so switch from the blocking state to the releasing state.

A trigger device for triggering an activation mechanism of a cannula assembly as described above comprises an actuator which is adapted to trigger, in particular move, the activation mechanism of the cannula assembly, and a control element. In accordance with one embodiment, the control element can be gripped by a user and thus manually operated by the user in order to initiate a triggering process for the actuator.

In accordance with one embodiment, the control element is adapted to be manually gripped by the user or to receive an operating effect for example from a medical or diabetes therapy device which is coupled to or integrated into or with the trigger device. The actuator is coupled to the control element in order to exert an activating effect when the control element is operated.

In one embodiment, the trigger device can be adapted to convert the user operation - such as touching, pushing, pulling or rotating the control element - into a mechanical and/or electromagnetic and/or thermal and/or hydraulic and/or pneumatic, externally supplied activating effect exerted on a blocking element of the activation mechanism in order to trigger, more specifically move, the blocking element.

The actuator can in particular comprise a release element adapted to be advanced outside the trigger device in order to interact with a retaining element of the activation mechanism for transferring or exerting a mechanical effect such as a shifting force on the retaining element.

The release element can in particular comprise one of the following:
- a shiftable trigger pin or shiftable release plug adapted to exert an outward linear force;
- a shiftable wedge-shaped element adapted to exert an outward lateral force;
- a magnet which can be moved and/or activated and is adapted to exert an outward, variable magnetic force;
- an electrical circuit, if for example a pyrotechnic device is used.

The trigger device can in particular comprise a trigger body or housing for the control element and actuator. The trigger body and control element can be designed to be basically identical. This embodiment can be implemented for example by a parallelepiped or cuboid or cylindrical body comprising an opening for accommodating the inserter module. The actuator can be embodied by one or more bar-shaped elements which protrude onto or into or contact the inserter module in order to exert an activation effect, such as a pushing effect on a blocking element of the activation mechanism.

Alternatively, the trigger body, control element and actuator can be designed to be basically identical. This embodiment can for example be implemented by a fluid connector of a fluid source or infusion pump which can be connected or attached to the inserter module, wherein the fluid connector can be adapted such that attaching or connecting the fluid connector to the inserter module exerts an activation effect such as a pushing effect on a blocking element of the activation mechanism, in order to trigger the activation mechanism of the cannula assembly.

The trigger device can in particular comprise a trigger interface which is adapted to transfer the activation effect or triggering information to the activation mechanism. In accordance with one embodiment, the trigger interface can be a mechanical interface comprising the actuator for transferring the activation effect, or an electrical or radio interface for transferring the triggering information.

The actuator and/or the control element can in particular be included or integrated in a medical or diabetes therapy device such as a glucose meter, or a fluid pump such as an infusion pump or an insulin pump. Optionally, the actuator and/or the control element can comprise the medical or diabetes therapy device.

The trigger device can in particular comprise key elements for interacting with a security mechanism of the cannula assembly which is adapted to be switched from a blocking state, in which the security mechanism prevents an energy store from being discharged, to a releasing state in which the energy store can be discharged. The key elements and the security mechanism can only interact if the key elements and the security mechanism fulfil certain matching criteria.

In accordance with one embodiment, the security mechanism and the blocking mechanism of the cannula assembly can be adapted so as to be cascaded or to operate in succession. The security mechanism can block the blocking mechanism against being released for as long as the matching criteria between the key elements and the security mechanism are not fulfilled.

In accordance with another aspect, the invention relates to a medical or diabetes therapy device such as a glucose meter or an infusion pump or fluid connector comprising a cannula system as described above. The medical or diabetes therapy device can be shaped as a cylinder or a box with a cross-section which is adapted to match a corresponding means of the activation mechanism. In accordance with one embodiment, the activation mechanism can only be triggered by a trigger device which matches the activation mechanism, thus fulfilling matching criteria. Such matching criteria can for example be that the physical form of the medical or diabetes therapy device is adapted to fit a correspondingly shaped inserter module. The trigger device or the medical or diabetes therapy device can comprise a security mechanism which is designed to match a security mechanism of an activation mechanism of a cannula assembly.

Optionally, an opening in the medical or diabetes therapy device which has a specific cross-section or profile can be designed such that it can only receive a correspondingly - more specifically, complementarily - shaped, for example bar-shaped inserter module which has a suitably shaped cross-sectional profile, wherein triggering is only possible when the inserter module is inserted into the opening of the medical or diabetes therapy device.

If matching criteria have to be fulfilled in order for triggering to be possible, this can substantially improve the operating security of the cannula assembly by reducing the risk of the cannula assembly being inadvertently or accidentally activated.

The invention relates to a cannula system comprising a cannula assembly as described above and a trigger device as described above.

Thus, the invention relates to a cannula system which is to be used with a fluid source or liquid supply or a pump for delivering a fluid or liquid to the cannula, such as an infusion pump. The cannula system comprises a disposable cannula assembly and a reusable trigger device.

The cannula assembly comprises an energy store and a cannula, wherein the cannula can be moved from a pre-operational state to an operational state using the energy of the energy store and the movement is triggered from outside the cannula assembly. The trigger device can be connected, such that it can be separated, to the cannula assembly or energy store in order to trigger the movement of the cannula.

In one embodiment, the trigger device can be distinct from the cannula assembly and/or from the energy store.

A trigger interface of the trigger device which comprises an actuator can in particular be designed to interact with a trigger interface of the cannula assembly in order to transfer and/or convert a trigger into a mechanical and/or electromagnetic and/or thermal and/or hydraulic and/or pneumatic activating effect for triggering an activation mechanism of the trigger device.

A release element of the trigger device which is designed to interact with a retaining element of the activation mechanism can in particular comprise one of the following elements: a shiftable trigger pin; a shiftable wedge-shaped element; a magnet which can be moved and/or activated; and a movable release plug.

The shiftable trigger pin can in particular be adapted to laterally push a box-shaped cap which is fixed to a retaining hook, thus pivoting the retaining hook in order to release it from the engagement with a bearing element.

The shiftable wedge-shaped element can be adapted to engage with a wedge-shaped cap which is fixed to a retaining hook, wherein the wedge-shaped element is adapted to be complementary to the wedge-shaped cap, thus pivoting the retaining hook in order to release it from the engagement with a bearing element.

The magnet which can be moved and/or activated can be adapted to engage with a ferromagnetic cap which is fixed to a retaining hook, thus pivoting the retaining hook in order to release it from the engagement with a bearing element.

The movable release plug can in particular be fixed to a fluid connector and adapted to engage with a levered retaining hook in order to switch from the blocking state to the releasing state.

A method for delivering a fluid or liquid to a cannula is disclosed herein. The method is applied to a cannula assembly comprising a cannula module and an inserter module and a trigger device which can be connected, such that it can be separated, to the cannula assembly. The inserter module comprises an energy store and an activation mechanism.

The method comprises the steps of: triggering the activation mechanism of the inserter module; moving the cannula from a pre-operational state to an operational state; and separating the trigger device from the cannula assembly. The activation mechanism is triggered by the trigger device, and this in turn enables the energy store to be discharged. The cannula is moved to an operational state using the energy of the energy store, for example by at least partially discharging the energy store.

The cannula system is suitably positioned on the surface or skin of the object or body before the triggering step is performed, wherein a suitable position for the cannula system includes a desired inclination towards the surface of the object or body.

The method can be performed without piercing the skin of a human or animal or in the absence of a human or animal body. The method can also be performed for the purpose of piercing the skin of a human or an animal.

In one embodiment, the energy store can be prevented from being discharged when the trigger device or a medical or diabetes therapy device comprising the trigger device is detached from the cannula assembly. Attaching the trigger device or the medical or diabetes therapy device comprising the trigger device to the cannula assembly enables the activation mechanism to be triggered, in order to discharge the energy store.

In accordance with one embodiment, the activation mechanism can only be triggered if matching criteria between key elements of the trigger device and a security mechanism of the cannula assembly are fulfilled.

In one embodiment, performing a triggering process of the trigger device can trigger the activation mechanism, for example by exerting a force, or can enable the activation mechanism to be triggered, for example by attaching the medical or diabetes therapy device to the cannula assembly or by transferring triggering information from the trigger device to the cannula assembly via an electrical or radio interface.

In accordance with one embodiment, the activation mechanism can be triggered by enabling the security mechanism of the cannula assembly to switch from a blocking state to a releasing state in order to permit the energy store to be discharged, wherein the matching criteria between the key elements of the trigger device and the security mechanism of the cannula assembly have to be fulfilled.

In one embodiment, a triggering process of the trigger device can induce an activating effect of the trigger device which is adapted to initialise the discharge of the energy store or to release at least one retaining element of the activation mechanism, more specifically of the security mechanism, in order to enable the energy store to be discharged.

In accordance with one embodiment, the discharge can be enabled by switching the security mechanism from a blocking state to a releasing state. It is only possible to discharge the energy store or to initialise the discharge in the releasing state of the security mechanism.

In one embodiment, triggering the activation mechanism can involve at least one of the following steps:
- attaching the trigger device, such that it can be separated, to the cannula assembly;
- attaching a fluid connector or a medical or diabetes therapy device comprising the trigger device, such that it can be separated, to the cannula assembly;
- operating a control element of the trigger device;
- channelling a fluid or liquid into the cannula assembly.

In one embodiment, discharging the energy store can be enabled by at least one of the following steps:
- shifting an actuator of the trigger device;
- activating a motor in order to shift an actuator of the trigger device;
- activating a magnetic field in order to shift a plunger of the trigger device;
- wetting a spongy element of the activation mechanism in order to move at least one retaining element by lowering the consistency of the spongy element;
- igniting a pyrotechnic device.

A method for delivering a fluid or liquid to a cannula is presented, using:
- a cannula assembly comprising a cannula module and an inserter module, wherein the inserter module comprises an energy store and an activation mechanism; and
- a trigger device which can be connected, such that it can be separated, to the cannula assembly;
   said method comprising the steps of:
   a) triggering the activation mechanism of the inserter module using the trigger device, thus enabling the energy store to be discharged;
   b) moving the cannula into an operational state using the energy of the energy store; and
   c) separating the trigger device from the cannula assembly.

In one embodiment, the energy store is prevented from being discharged when the trigger device is detached from the cannula assembly, and attaching the trigger device to the cannula assembly enables the activation mechanism to be triggered, in order to discharge the energy store.

In one embodiment, performing a triggering process of the trigger device (triggers the activation mechanism or enables the activation mechanism to be triggered.

In one embodiment, a triggering process of the trigger device initialises the discharge of the energy store or releases at least one blocking element of the activation mechanism in order to enable the energy store to be discharged.

In one embodiment, triggering the activation mechanism involves at least one of the following steps:
- attaching the trigger device to the cannula assembly or energy store;
- attaching a fluid connector or a medical device or diabetes therapy device comprising the trigger device to the cannula assembly;
- operating a control element of the trigger device;
- channelling a fluid or liquid into the cannula assembly; and/or
wherein discharging the energy store is enabled or initiated by at least one of the following steps:
- shifting an actuator of the trigger device, manually or motor-driven, in order to exert a releasing force on a blocking element;
- wetting a spongy element of the activation mechanism in order to move at least one blocking element by lowering the consistency or structural strength of the spongy element;
- activating an ignition device in order to ignite a pyrotechnic device.

The accompanying drawings, which are incorporated in and constitute a part of this specification, illustrate several embodiments of the invention and, together with the description, serve to explain the principles of the invention. There is shown:
- Figure 1: a cannula assembly;
- Figure 2: a cannula module;
- Figure 3: an inserter module;
- Figure 4: a cannula system comprising a trigger device integrated in a medical or diabetes therapy device, wherein a mechanical activating effect is exerted by a shiftable pin which can be moved using an electromagnet;
- Figure 5: a cannula system comprising a trigger device integrated in a medical or diabetes therapy device, wherein a mechanical activating effect is exerted by a shiftable pin which can be moved manually using a push button;
- Figure 6: a cannula system comprising a trigger device integrated in a medical or diabetes therapy device, wherein a mechanical activating effect is exerted by an element which can be moved vertically and is wedge- shaped on one side;
- Figure 7: a cannula system comprising a trigger device integrated in a medical or diabetes therapy device, wherein a mechanical activating effect is exerted by a wedge-shaped element which can be moved vertically;
- Figure 8: a cannula system comprising a trigger device integrated in a medical or diabetes therapy device, wherein a magnetic activating effect is exerted by a magnet which can be moved;
- Figure 9: a cannula system comprising a trigger device integrated in a medical or diabetes therapy device, wherein a mechanical activating effect is exerted by a motor-driven shiftable pin;
- Figure 10: a cannula system comprising a trigger device integrated in a medical or diabetes therapy device, wherein an electric field which activates an ignition device is applied in order to trigger a pyrotechnic device;
- Figure 11: a cannula system comprising a trigger device integrated in a medical or diabetes therapy device, wherein the trigger device comprises key Figures 11A to 11F elements which match a security mechanism of the cannula assembly and wherein a mechanical activating effect is exerted by a wedge- shaped element which can be moved vertically; an insertion sequence using the cannula system shown in Figure 11;
- Figure 12A: a cannula system comprising a trigger device integrated in a medical or diabetes therapy device, wherein the triggering effect is exerted via a mechanical interface;
- Figure 12B: a cannula system comprising a trigger device integrated in a medical or diabetes therapy device, wherein the triggering effect is exerted via a radio interface;
- Figure 13: a cannula system comprising a trigger device integrated in a fluid connector;
- Figures 13A to 13E: an insertion sequence using the cannula system shown in Figure 13;
- Figure 14A: a cannula system in a pre-operational state, wherein triggering is effected by a fluid or liquid acting on a spongy element;
- Figure 14B: the cannula system of Figure 14A, in an operational state;
- Figure 15A: a cannula system in a pre-operational state, wherein triggering is effected by a fluid or liquid acting on an elastic element;
- Figure 15B: the cannula system of Figure 15A, in an operational state;
- Figure 16A: a first embodiment of a cannula system in a pre-operational state, wherein triggering is effected by a fluid or liquid acting on a shiftable plug;
- Figure 16B: the cannula system of Figure 16A, in an operational state;
- Figure 17A: a second embodiment of a cannula system in a pre-operational state, wherein triggering is effected by a fluid or liquid acting on a shiftable plug; and
- Figure 17B: the cannula system of Figure 17A, in an operational state.

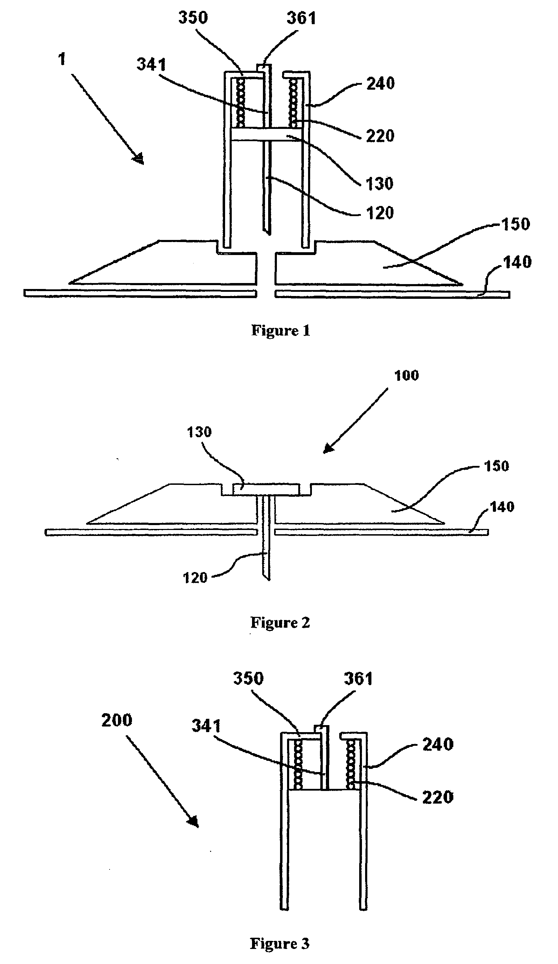

Figure 1 shows a cannula assembly 1 to be used in the invention. The cannula assembly 1 comprises a cannula module 100 as shown in Figure 2 and a disposable inserter module 200 as shown in Figure 3.

The cannula module 100 comprises an adhesive layer 140 which is to be placed on a user's skin. The cannula module 100 also comprises a cannula 120 consisting of a hollow structural element which remains in the tissue for the duration of use and through which the drug can be administered. This can be a hollow steel cannula with a pointed tip or a flexible tube, in particular made of Teflon. In the latter case, a guidance needle is provided inside the cannula tube. The guidance needle has a pointed tip and is slightly longer than the flexible cannula, but is generally not hollow. The guiding needle is required for piercing the skin and for stabilising the flexible cannula tube during insertion. Once insertion is complete, it is retracted and removed either manually or by a spring.

The cannula 120 is configured to assume two states: a pre-operational or initial state and an operational or final state. In the pre-operational state, which is shown in Figure 1, the cannula 120 is retracted relative to a skin-contacting surface 140. In the operational state, which is shown in Figure 2, the cannula is advanced relative to the skin-contacting surface 140 and projects from the skin-contacting surface 140.

The disposable inserter module 200 is designed to be releasably coupled to the cannula module 100 in the pre-operational state of the cannula 120, which is shown in Figure 1, and to be separated from the cannula module 100 in the operational state of the cannula 120, which is shown in Figure 2. The inserter module 200 comprises a biased spring 220, which is adapted to transform stored energy into kinetic energy when discharged, and an activation mechanism 341, 350, 361.

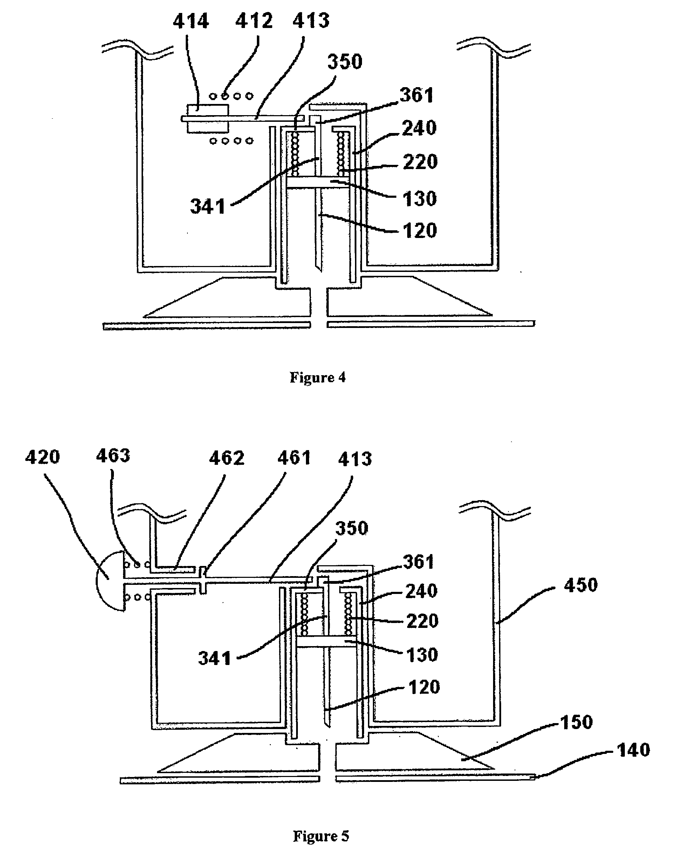

The biased spring 220 can be prevented from being discharged by blocking means 341, 350, wherein releasing the blocking means 341, 350 can enable the biased spring 220 to be discharged and the cannula 120 to be moved. The activation mechanism 341, 350, 361 can be triggered from outside the cannula assembly by a trigger device 411-463, which is shown in Figures 4 to 17. Triggering involves transferring a force from the trigger device 411-463 to the activation mechanism 341, 350, 361 in order to release the blocking means 341, 350, in order to advance the cannula 120.

The blocking means or retaining elements 341, 350 comprise a retaining hook 341 and a bearing element 350 which are designed to assume a blocking state in which the retaining hook 341 is releasably engaged with the bearing element 350, and a releasing state in which the retaining hook 341 is released from the bearing element 350. The biased or tensioned spring 220 is prevented from being discharged or relaxed in the blocking state, and the biased spring 220 is allowed to be discharged or relaxed in the releasing state.

The retaining hook is a pivoting retaining hook 341 comprising a hooked end which is releasably engaged with the bearing element 350, and an opposite, straight end which pivots on and is axially fixed to a plunger 130, wherein the pivoting retaining hook 341 can be released from the bearing element 350 by a lateral force which pushes against a box-shaped cap 361 on the hooked end of the retaining hook 341 and moves the retaining hook 341 from the blocking state to the releasing state.

Figures 4 to 12 show embodiments of a cannula system according to the invention, and in which the trigger device 411-463 is integrated in a medical or diabetes therapy device 450 such as a glucose meter or an insulin pump. Thus, the medical or diabetes therapy device 450 becomes the key to triggering the movement of the cannula 120. Alternatively, the cannula assembly 1 can be controlled and/or operated with or without mechanical contact between the cannula assembly 1 and the trigger device 411-463. In this document, the term "medical or diabetes therapy device" 450 is a device used by a person in diabetes therapy such as a glucose meter or an insulin pump.

As shown in Figure 4, a shiftable pin 413 and an electromagnet 412 are provided in the trigger device 411-463 for triggering the insertion of the cannula. If the electromagnet 412 is energised, the shiftable pin 413 moves to the right and interacts with the box-shaped cap 361 on the retaining hook 341, thus deflecting the retaining hook 341. Consequently, the spring 220 is released and acts on the plunger 130, to which the infusion cannula 120 is attached. The plunger 130 and the spring 220 move downwards towards the patient's skin, such that the infusion cannula 120 is inserted into the tissue. The shiftable pin 413 is moved back into its initial position by another spring (not indicated) in the trigger device 411-463.

The movement of the infusion cannula 120 is limited by the plunger 130 contacting a cannula hub 150 when the infusion cannula 120 is fully inserted. The cannula hub 150 is attached to the adhesive layer 140 and also comprises the fluid connector to the medical or diabetes therapy device (not shown).

Once insertion is complete, the trigger device 411-463 is removed, followed by the inserter module 200.

Various modifications to these embodiments of the invention are possible. For example, the shiftable pin 413 shown in Figure 4 could also be operated manually by the user via a push button 420 included in the trigger device 411-463, as shown in Figure 5.

In Figures 4 to 11, the medical or diabetes therapy device 450 is attached to the cannula assembly 1 in order to initiate an insertion of the cannula. Insertion is triggered by means of a control element 420, as shown in Figure 5.

In Figure 4, moving a piston rod or ferrite rod 414 by means of a coil 412 can move the pivoting retaining hook 341 until it is completely released and the cannula 120 is then injected. The energy store is a spring 220 which is held in a compressed state and released in order to insert the infusion cannula 120. The retaining hook 341 interacts - prior to the insertion of the cannula - with a corresponding element (not indicated) of the inserter module 200 in order to hold the spring 220 in the compressed state and the infusion cannula 120 in the pre-operational state.

In Figure 5, pushing a button 420 moves the pivoting retaining hook 341 until it is released. Instead of one button, it is also possible to use two buttons and two pivoting retaining hooks 341 (not shown). The insertion of the cannula is then triggered only if both buttons are pushed.

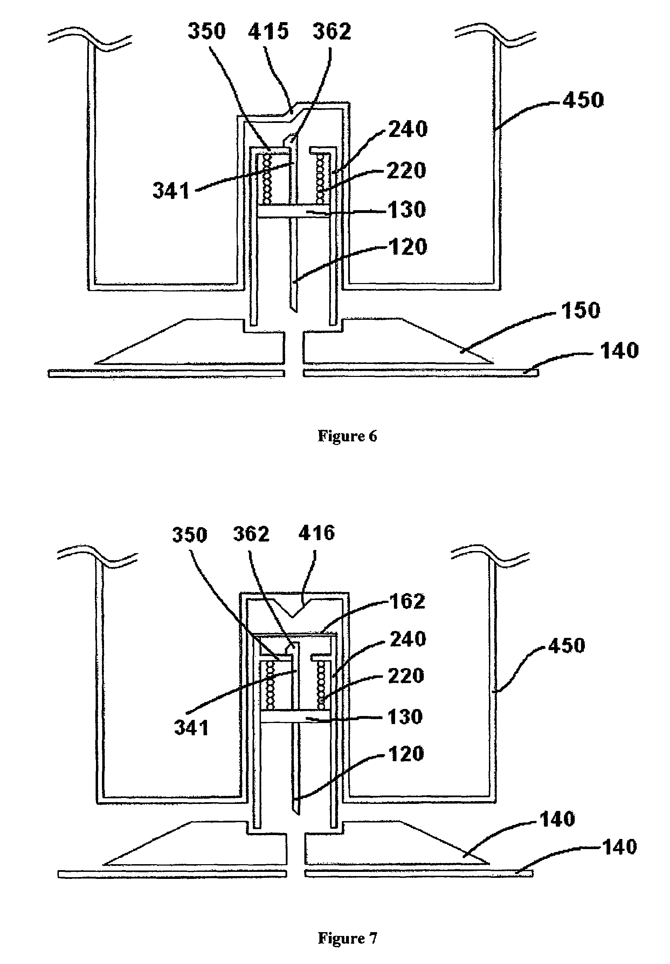

In Figure 6, the pivoting retaining hook 341 is moved laterally by means of an inclined wedge-shaped surface on one side of a wedge-shaped element 415, until the retaining hook 341 is released.

In Figure 7, a prism-shaped or wedge-shaped element 416 is moved towards the retaining hook 341 until the retaining hook 341 is released and the cannula 120 is then injected. The prism 416 pierces an initially sterile barrier such as a septum 162 which covers the trigger device 411-463.

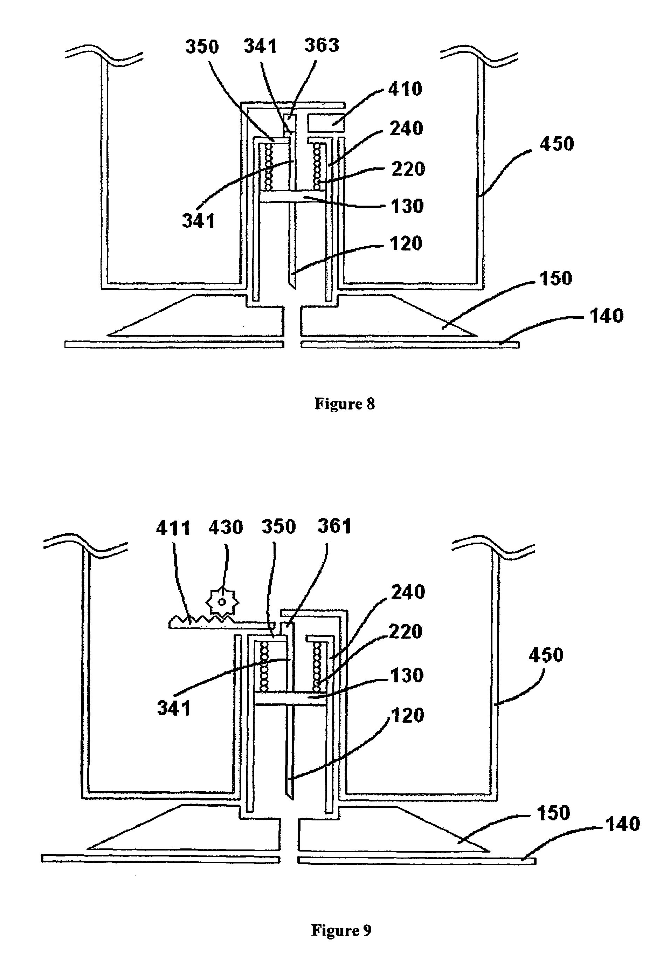

Figure 8 shows a cannula assembly 1 comprising a pivoting retaining hook 341, the upper side of which comprises a snapping element or a metal element or a ferromagnetic cap 363 which holds the spring 220 in its biased state. A permanent magnet 410 which is integrated into the device is moved towards the retaining hook 341 until the retaining hook 341 is released and the cannula 120 is injected.

In Figure 9, a motor-driven gear rack 411 moves the pivoting retaining hook 341 until it is released and the cannula 120 is injected.

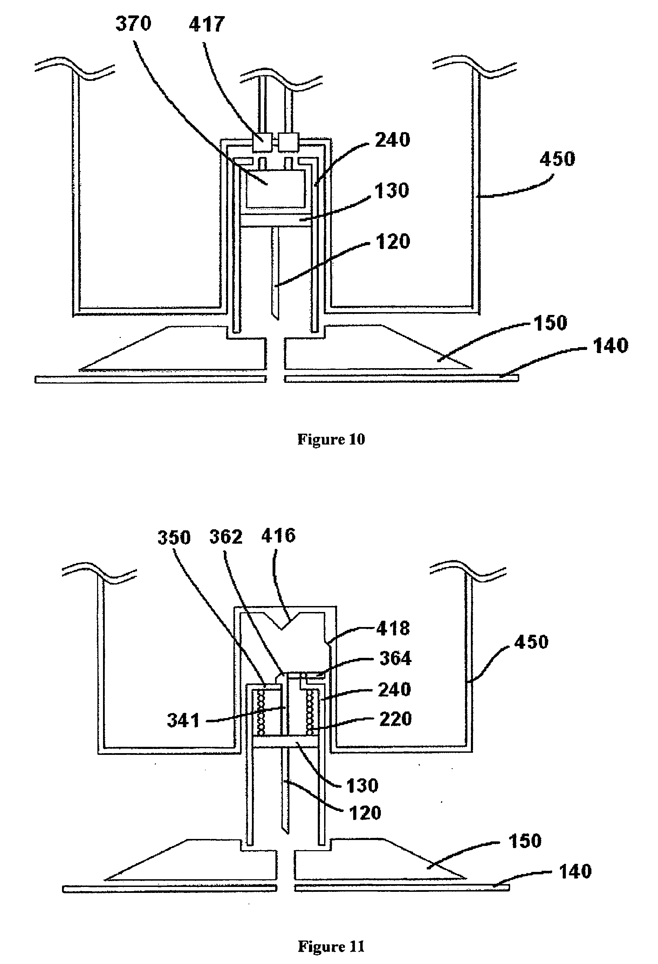

Figure 10 shows another example embodiment, which does not use a spring as the energy store. Instead, the energy store is realised as a pyrotechnic device 417 which releases stored chemical energy in a controlled "explosion" upon receiving an electrical trigger signal via trigger contacts in the trigger device 411-463. The corresponding control and power circuitry for triggering the pyrotechnic device 417 is included in the trigger device 411-463.

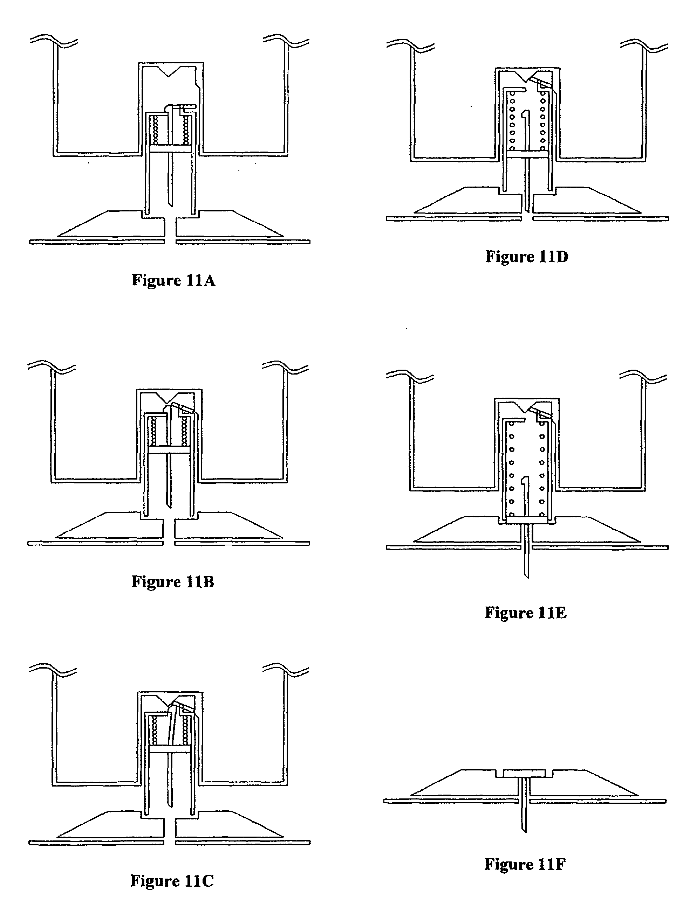

In Figure 11, a prism-shaped or wedge-shaped element 416 is moved towards the retaining hook 341 until the retaining hook 341 is released and the cannula 120 is then injected.

The cannula assembly comprises a security mechanism 341, 362, 364 which is adapted to switch from a blocking state in which the security mechanism 341, 362, 364 prevents the spring 220 from discharging, for example when the trigger device 411-463 is detached from the cannula assembly, to a releasing state in which the spring 220 can be discharged. The security mechanism 341, 362, 364 comprises a retaining hook 341 and a retaining pin 364 which can be levered on a fulcrum from the blocking state, in which the retaining pin 364 prevents the retaining hook 341 from pivoting, to the releasing state in which the retaining hook 341 is released for pivoting.

The top of the retaining hook 341 comprises a wedge-shaped cap 362 which is designed to facilitate an engagement between the retaining pin 364 and the retaining hook 341 in the blocking state and to enable an engagement between a wedge-shaped element 416 and the retaining hook 341 in order to release the retaining hook 341 from its engagement with a bearing element 350 and so enable the spring 220 to be discharged. The retaining pin 364 can be levered on the fulcrum from the blocking state to the releasing state by a release buckle 418 of the trigger device 411-463, wherein the release buckle 418 and the retaining pin 364 are designed to match, such that advancing the trigger device 411-463 or the medical or diabetes therapy device 450 comprising the trigger device 411-463 from a distal position with respect to the skin of the patient enables the retaining pin 364 to be levered.

The security mechanism 341, 362, 364 is designed in a similar way to a locking device, wherein the medical or diabetes therapy device 450 which comprises the release buckle 418 represents the key which can unlock the security mechanism 341, 362, 364. This ensures that the retaining hook 341 is not inadvertently released. Accordingly, the security mechanism 341, 362, 364 can be designed in a similar way to a mechanical lock. The aim of the security mechanism 341, 362, 364 is to prevent an insertion of the cannula from being triggered inadvertently or through deliberate misuse. Triggering is only possible using the corresponding counterpart or key, for example the release buckle 418 in the medical or diabetes therapy device 450.

Figures 11 A to 11F show an insertion sequence using the cannula system shown in Figure 11.

In Figure 11A, the inserter module 200 has been introduced into the corresponding opening of the medical or diabetes therapy device 450. The retaining hook 341 is still in engagement with the bearing element 350, and the retaining pin 364 is still in engagement with the wedge-shaped cap 362 of the retaining hook 341.

In Figure 11B, a curved profile or release buckle 418 which is part of the opening of the medical or diabetes therapy device 450 tilts the locking lever or levered retaining pin 364. Thus, the pivoting retaining hook 341 is no longer blocked or secured.

In Figure 11C, the inserter module 200 passes further into the corresponding opening, and the pivoting retaining hook 341 is displaced by the prism-shaped element 416 and thus released.

In Figure 11D, releasing the pivoting retaining hook 341 releases the lock on the needle assembly or cannula 120. The spring 220 can relax and move the cannula 120 towards the patient.

In Figure 11E, the cannula 120 is advanced into its final position in the cannula hub 150. In this position, the needle assembly or cannula 120 snaps into the cannula hub 150 (not explicitly shown). The inserter module 200 is also released from the cannula hub 150 (not shown).

In Figure 11F, the inserter module 200 has been removed and the cannula module 100 is ready to be attached to a fluid or liquid tube of a fluid source such as an infusion pump.

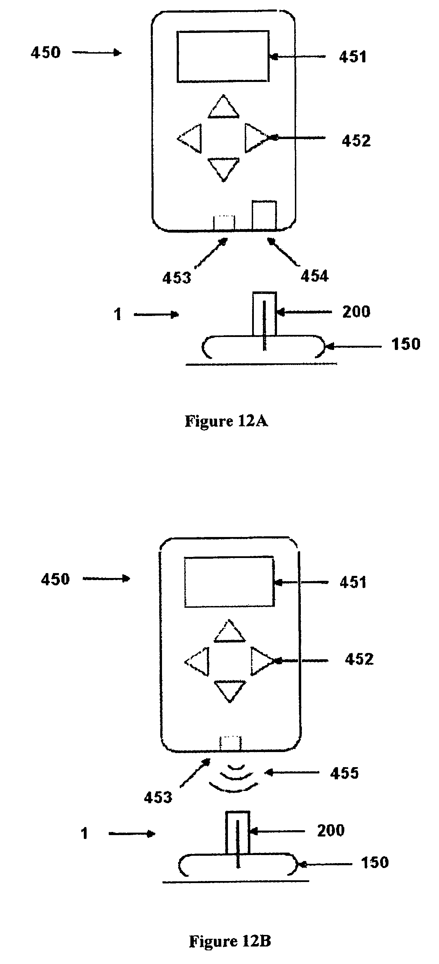

Figure 12A shows an example embodiment of a system comprising a cannula assembly 1 and a blood glucose meter 450 comprising an integrated trigger device 411-463. Alternatively, the trigger device 411-463 can be integrated into an insulin pump or other diabetes therapy device. Inserting the cannula assembly 1 into a slot of the blood glucose meter 450 which features a mechanical or electrical trigger interface 454 triggers the cannula assembly 1.

Figure 12B shows a system similar to that in Figure 12A, but in which the cannula assembly 1 is triggered via a radio trigger interface 455.

The embodiments shown in Figures 4 to 12 have numerous advantages, a number of which are discussed below.

All the mechanical components, which are susceptible to abrasive wear, are integrated into the disposable inserter module 200 rather than the medical or diabetes therapy device 450. The inserter module 200 can be designed for a small number of applications or for only one application, thus drastically reducing its mechanical complexity. This also substantially reduces the chances of the inserter module 200 malfunctioning due to abrasive wear.

The medical or diabetes therapy device 450 comprises the trigger device 411-463, which can be small in volume. Therefore, the medical or diabetes therapy device 450 does not become significantly larger or more expensive.

In some embodiments, the disposable inserter module 200 does not have any controls. Without the inclusion of controls, the volume of the disposable inserter module 200 can be reduced even further.

Controlling the insertion of the cannula using a medical or diabetes therapy device 450 can increase the operating security. The danger of accidentally triggering the cannula assembly 1 is reduced, because the cannula assembly 1 can only be triggered by interacting with the medical or diabetes therapy device 450 and not by directly controlling the inserter module 200.

Additional software functions can easily be integrated, for example a function whereby the insertion of the cannula 120 is triggered at a random point within a several-second period or e.g. 10-second period, such that the patient is surprised and feels less pain.

Each triggering or insertion procedure can be recorded, together with time and date stamps, on the diabetes therapy device such as the glucose meter or insulin pump. This allows the duration of use of the infusion cannula to be better monitored and managed.

Additional data, such as for example the insertion location, can also be captured for each triggering or insertion procedure. Thus, the medical or diabetes therapy device 450 can aid the patient in appropriately distributing the insertion locations evenly over the body.

The cannula assembly 1 is very easy to handle. If the trigger device 411-463 is initially inserted or integrated into a medical or diabetes therapy device 450, it is easier to manipulate these devices instead of a small infusion set.

The embodiments in Figures 4 to 12 have a mechanical interface or a non-contact or inductive or magnetic interface, wherein the trigger device 411-463 can be integrated into a medical or diabetes therapy device 450.

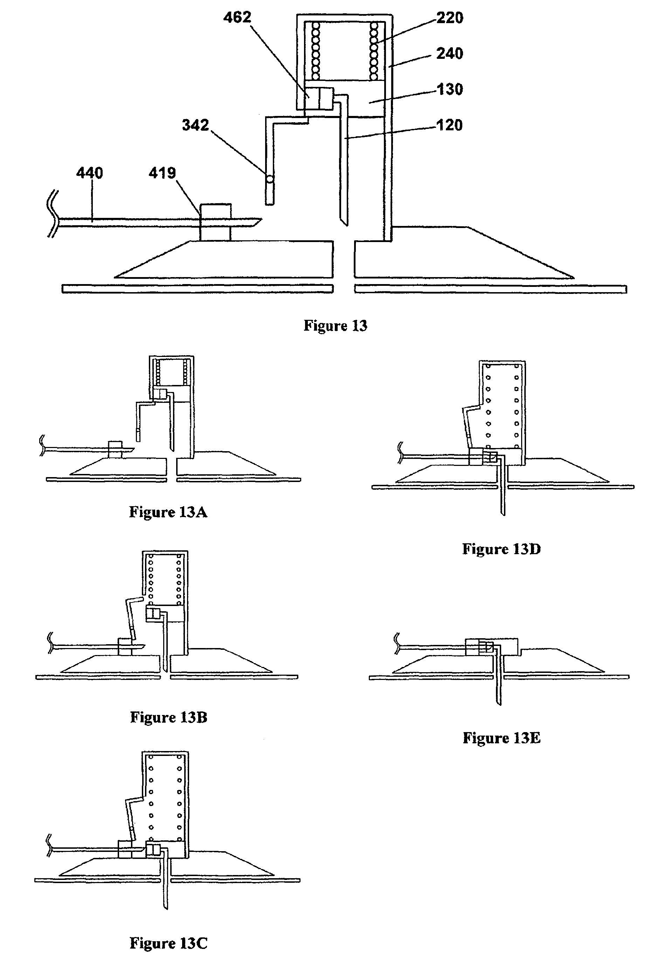

Figure 13 shows an inserter module which is triggered without using control elements, but rather by connecting a fluid connector 440 to the cannula hub 150.

The patient positions a cannula assembly 1, comprising an integrated inserter module 200, on the skin and then attaches the fluid connector 440, which has been filled with insulin beforehand, to the cannula hub 150. This triggers the insertion of the cannula 120. The inserter module 200 is then decoupled and/or detached from the cannula hub 150. The user can then dispose of the inserter module 200 and begin an insulin infusion procedure.

The inserter module is a disposable inserter module 200 comprising a spring 220. The spring 220 can either be biased and integrated into the inserter module 200 or tensed manually by the user before triggering is initiated.

The inserter module 200 has a mechanical locking/releasing mechanism which initially holds the spring 220 securely in a tensioned state. Once a fluid connector 440 is connected to the cannula assembly 1, which has already been attached to the user's skin, the locking/releasing mechanism is triggered and a movable needle assembly or cannula 120 is applied to the skin. The locking/releasing mechanism comprises a slider or release plug 419 and a tilting device such as a levered retaining hook 342. Its mechanical function is to convert a linear movement of the fluid connector 440, as it connects with the cannula hub 150, into a mechanical movement which releases a blocking element and thus causes the biased spring 220 to be discharged. Figure 13 shows a cannula assembly 1 which employs a tilting mechanism 342 as the locking/releasing mechanism. The interaction between the release plug 419 and the retaining hook 342 causes the retaining hook 342 to tilt or pivot, thus allowing the spring 220 to be discharged.

The trigger device 411-463 is integrated with the tubing connector 440 which fluidically connects the cannula 120 to the infusion pump and is connected to the cannula assembly 1 via a tubing connection after the cannula assembly 1 has been attached to the skin. The tubing connector 440 includes a connector cannula for piercing a septum 462.

Figures 13A to 13F show an insertion sequence using the cannula system shown in Figure 13.

In Figure 13A, the spring 220 is initially held in a compressed state by a levered retaining hook 342. The retaining hook 342 also holds the plunger 130, together with the infusion cannula 120, in a pre-operational (retracted) state. The tubing connector 440 is not yet in engagement with the cannula assembly 1. The release plug 419 of the tubing connector 440 is moved linearly to the right and comes into contact with the retaining hook 342.

In Figure 13B, continuing the movement of the tubing connector 440 causes the retaining hook 342 to be levered in an anti-clockwise direction about its levering point, thereby releasing the engagement between the retaining hook 342 and the plunger 130. Accordingly, the plunger 130 together with the infusion cannula 120 is moved downwards, towards the skin, by the spring 220.

In Figure 13C, the plunger 130 comes into contact with the cannula hub 150, and the cannula is then in the operational state. The plunger 130 has an integrated septum 462 for fluidically connecting the infusion cannula 120. In the operational state, the septum 462 is aligned with the tubing connector 440 and the cannula 120, and the spring 220 is in a relaxed end position. The cannula 120 is secured on the cannula hub 150 by means of a snapping mechanism (not shown).

In Figure 13D, the linear movement of the tubing connector 440 is continued, and the connector cannula pierces the septum 462, thus establishing a fluidic connection between the tubing connector 440 and the infusion cannula 120. The tubing connector 440 and the cannula hub 150 advantageously engage via a latching mechanism (not shown).

In Figure 13E, the inserter module 200 is removed and the delivery of insulin can be started.

In the description, the cannula 120 is a hollow steel needle with a pointed tip. The invention also however relates to infusion using a flexible tube. The flexible tube is then mechanically supported during insertion by a guiding needle. In embodiments in which a guiding needle is provided, the guiding needle is retracted manually or by a second spring, once insertion is complete.

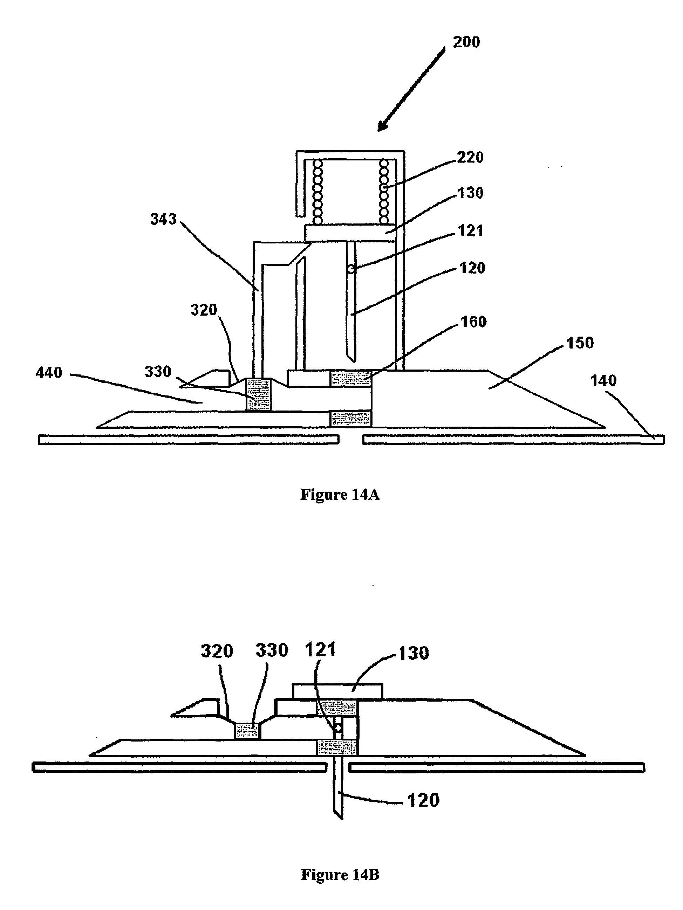

Figures 14 to 17 show a cannula system in which the cannula assembly is triggered by the flow of a medical product or liquid such as insulin into the activation mechanism.

The patient first performs a priming procedure, i.e. fills the fluid connector 440 and the connection tube to the pump with fluid, and attaches a cannula hub 150 comprising an integrated inserter module 200. The user then sets their individual insulin dose and places the cannula assembly 1 onto the skin and activates an "Insert" function on the insulin pump. Alternatively, the user first places the cannula assembly 1 onto the skin, activates the "Insert" function on the insulin pump and then sets their individual insulin dose. After a few seconds, the cannula 120 is inserted. The user then sets their individual insulin dose and starts the fluid or liquid transport. The pump is typically already programmed such that the pump merely has to be started. In principle, this can also be performed automatically at the end of the priming procedure.

Figures 14A and 14B show a cannula system in which the cannula assembly is triggered by a fluid acting on a spongy element 330. The cannula system comprises a disposable inserter module 200 featuring a biased spring 220. Figure 14A shows the cannula system in a pre-operational state of the cannula 120, and Figure 14B shows the cannula system in the operational state of the cannula 120.

A levered retaining hook 343 is laterally provided and holds the spring 220 in the biased state. Inserting the fluid connector 440 levers the retaining hook 343, and the cannula 120 is then injected. The fluid connector 440 also locks the cannula 120 in place.

When the fluid supplied, for example insulin, acts on the spongy element 330, the spongy element 330 becomes soft and a biased elastic element or flexible seal 320 made of a material such as silicone rubber is inverted. This triggers the activation or insertion mechanism. The retaining hook 343 and a rigid vertical bar (not provided with a separate reference sign) are chamfered. The rigid bar thus bends the retaining hook 343 to the left, allowing the plunger 130 to move downwards and pass the retaining hook 343 without being blocked.

The user places the cannula assembly 1 together with the integrated inserter on the skin and then attaches the fluid connector 440, which has been filled with insulin beforehand, to the cannula hub 150. Then the user activates the insulin delivery decive or pump and starts the insulin flow and thus triggers the insertion of the cannula, after which the inserter module 200 is decoupled from the cannula hub 150. The user can dispose of the inserter module 200 and start an insulin infusion procedure.

In practice, the priming procedure can be carried out in three steps: (i) the tubing and the fluid connector 440 are primed, in a detached state, in a first priming step; (ii) the fluid connector 440 is attached; (iii) a second priming procedure is carried out in order to fill the cannula with insulin e.g. after activating the "Insert" function of the insulin pump. Only in step (iii) is the spongy element wetted and the insertion of the cannula triggered. The amount of insulin employed in the second priming procedure should not exceed the amount needed to fill the cannula, in order to prevent additional insulin being unintentionally administered to the patient.

In this embodiment, the insertion of the cannula is triggered by the insulin which flows into the cannula assembly 1 from the insulin pump, wherein the insulin pump serves as the trigger device 411-463.

In the pre-operational state, the spring 220 is held in a compressed state by a retaining hook 343, as described above. The retaining hook 343 is supported by the elastic element or flexible seal 320 which is in turn connected to the spongy element 330 in the fluid channel or connector 440. When insulin flows into the fluid connector 440 of the cannula module 100, the spongy element 330 is structurally weakened, such that it can no longer support the elastic element or flexible seal 320. Accordingly, the spongy element 330 causes the elastic element or flexible seal 320 to change its state, such that the engagement between the plunger 130 and the retaining hook 343 is released.

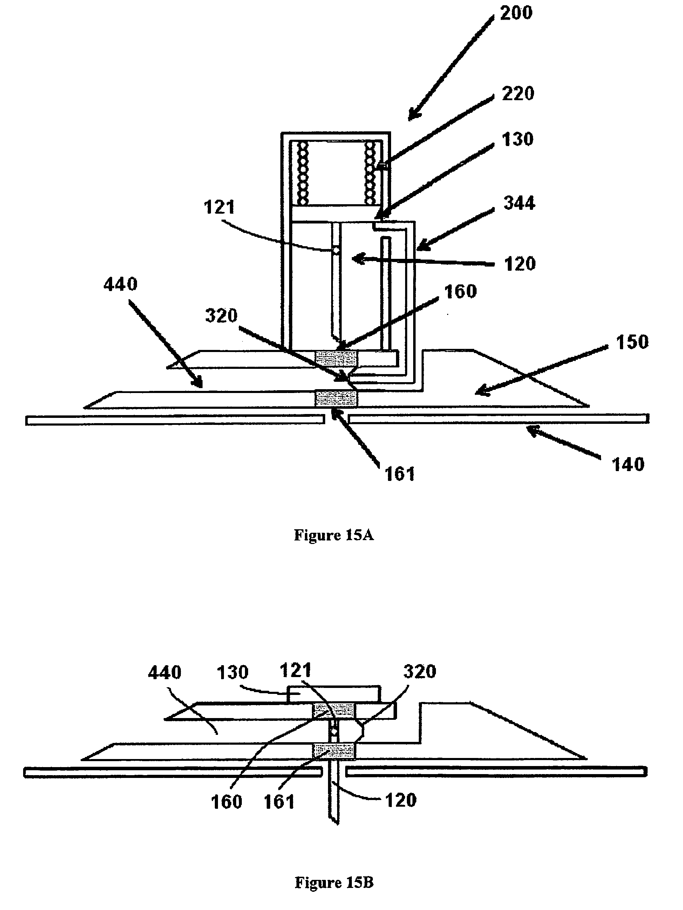

Figures 15A and 15B show another embodiment of the invention in which the cannula assembly is triggered by a fluid or liquid acting on an elastic element 320 which can be designed as a bistable elastic element. Once insulin has been transferred into the cannula hub 150 and a certain pressure has accumulated, the elastic element 320 is inverted outwards and thus triggers the inserter module 200. Figure 15A shows the cannula system in the pre-operational state of the cannula 120, and Figure 15B shows the cannula system in the operational state of the cannula 120.

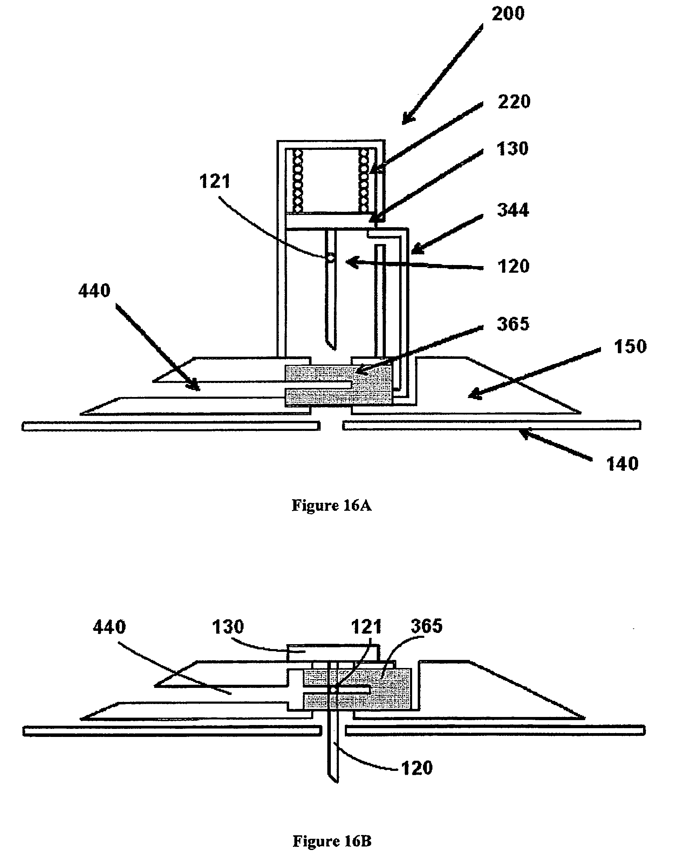

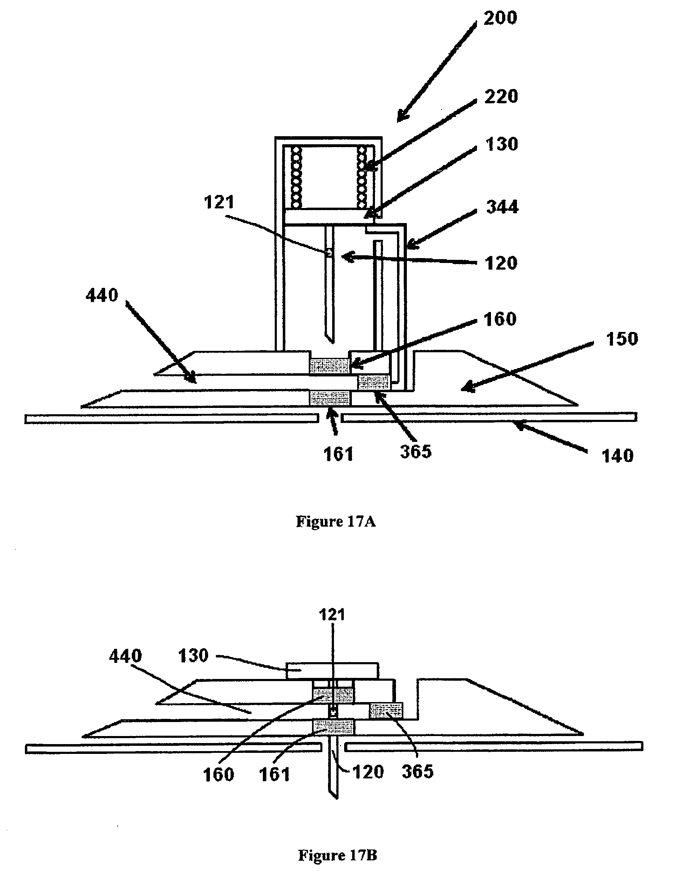

Figures 16 and 17 show additional embodiments of the invention in which the cannula assembly is triggered by a fluid or liquid acting on a shiftable plug 365. Once insulin is transferred into the cannula hub 150, a stopper or shiftable plug 365 is moved and triggers the inserter module 200 by releasing the lock on the biased spring 220, which in turn moves the cannula 120. The quantity of insulin which is transferred in the course of the "Insert" function corresponds to the filling volume of the fluid channel 140 and cannula 120.

Figures 16A and 17A show the cannula system in the pre-operational state of the cannula 120, and Figures 16B and 17B show the cannula system in the operational state of the cannula 120.

As shown in Figures 14 to 17, the cannula 120 comprises a lateral opening 121 which in the operational state of the cannula 120 is positioned so as to be in fluid connection with the fluid connector 440, such that the fluid or liquid can flow through the fluid connector 440, via the lateral opening 121 of the cannula 120, into the cannula 120.

### Reference signs

- 1: cannula assembly
- 100: cannula module
- 120: cannula, infusion cannula
- 121: lateral opening of the cannula
- 130: plunger
- 140: skin-contacting surface, adhesive layer
- 150: cannula hub
- 160: first septum
- 161: second septum
- 162: septum
- 200: inserter module
- 220: energy store, spring
- 240: inserter body
- 320: elastic element, bistable elastic element, flexible seal
- 330: spongy element
- 341: pivoting retaining hook
- 342: levered retaining hook
- 343: axially shiftable retaining hook
- 344: laterally shiftable retaining hook
- 350: bearing element, retaining element, retaining bearing
- 361: box-shaped cap
- 362: wedge-shaped cap
- 363: ferromagnetic cap
- 364: levered retaining pin
- 365: shiftable plug
- 370: pyrotechnic device
- 410: magnet
- 411: gear rack
- 412: coil, electromagnet
- 413: shiftable pin
- 414: piston rod, ferrite rod
- 415: wedge-shaped element, element which is wedge-shaped on one side
- 416: wedge-shaped element
- 417: ignition device
- 418: release buckle
- 419: release plug
- 420: push button
- 430: gear wheel
- 440: fluid connector, tubing connector
- 450: medical device or diabetes therapy device, glucose meter, insulin pump
- 451: display
- 452: control elements
- 453: test strip slot
- 454: mechanical trigger interface, electrical trigger interface
- 455: radio trigger interface
- 461: latching pin
- 462: septum
- 463: compression spring

## Claims

1. A cannula system, to be used with a fluid source or liquid supply such as an infusion pump for delivering a fluid or liquid to the cannula (120), said cannula system comprising:
- a disposable cannula assembly (1) comprising a skin-contacting surface (140) to be placed on an outer surface of an object or the skin of a body, an energy store (220) and a cannula (120) which can be moved from a pre-operational state, in which the cannula (120) is retracted such that it does not project from the skin-contacting surface (140), to an operational state, in which the cannula (120) projects from the skin-contacting surface (140), using the energy of the energy store (220), wherein the movement is triggered from outside the cannula assembly (1); **characterized by**
- a reusable trigger device (411-463) which can be connected, such that it can be separated, to the cannula assembly (1) or energy store (220) in order to trigger the movement of the cannula (120).

2. The cannula system according to claim 1, wherein the trigger device (411-463) is distinct from the cannula assembly (1) and/or from the energy store (220).

3. The cannula system according to claim 1 or 2, wherein the cannula assembly (1) comprises a cannula module (100), comprising the skin-contacting surface (140) and the cannula (120), and an inserter module (200) comprising the energy storage (220) and an activation mechanism (320, 370) which can be triggered from outside the cannula assembly (1) by the trigger device (411-463) and is adapted to prevent the energy store (220) from being discharged before it is triggered by the trigger device (411-463), thus maintaining the pre-operational state of the cannula (120), and is adapted to enable the energy store (220) to be discharged after it is triggered by the trigger device (411-463), thus forcing the cannula (120) into the operational state.

4. The cannula system according to the preceding claim, wherein the inserter module (200) is designed to be releasably coupled to the cannula module (100).

5. The cannula assembly according to claim 3 or 4, wherein a blocking element (320-365) is provided in order to prevent the energy store (220) from being discharged and can be released from a blocking position in order to enable or cause the energy store (220) to be discharged.

6. The cannula system according to claim 5, wherein the activation mechanism (320-370) comprises a trigger interface (320-344) which is adapted to receive an external trigger signal and to convert the trigger signal into an activating effect which acts on the blocking element (320-365) or on an activating element (370) in order to enable or cause the energy store (220) to be discharged.

7. The cannula system according to claim 6, wherein the activating effect is a mechanical and/or magnetic and/or electric and/or electromagnetic and/or hydraulic and/or thermal and/or pneumatic and/or pyrotechnic activating effect.

8. The cannula system according to any one of claims 3 to 7, wherein the activation mechanism (320-370) comprises a security mechanism (364) which is adapted to switch from a blocking state in which the security mechanism (364) prevents the activation mechanism (320-370) from being triggered or the blocking mechanism (320-365) from being released, thus preventing the energy store (220) from being discharged, to a releasing state in which the energy store (220) can be discharged or in which it is possible to trigger or cause the energy store (220) to be discharged.

9. The cannula system according to any one of claims 5 to 8, wherein:
the activation mechanism (320-370) comprises a blocking element (341-344) and a retaining element (350);
the blocking element (341-344) is releasably attached to the retaining element (350) in order to prevent the energy store (220) from being discharged;
the blocking element (341-344) is adapted to be detached from the retaining element (350) by the activating effect, which causes the energy store (220) to be discharged; and
the activation mechanism (320-370) can be triggered by moving the blocking element (341-344).

10. The cannula system according to any one of claims 1 to 9, wherein the trigger device (411-463) is a part of a medical device or diabetes therapy device, such as a glucose meter (450) or an infusion pump or fluid connector (440).

## Patentansprüche

1. Kanülensystem, das mit einer Fluidquelle oder einer Flüssigkeitsversorgung wie etwa einer Infusionspumpe zu verwenden ist, um ein Fluid oder eine Flüssigkeit an die Kanüle (120) abzugeben, wobei das Kanülensystem umfasst:
- eine wegwerfbare Kanülenanordnung (1) mit einer Hautkontaktoberfläche (140), die auf einer äußeren Oberfläche eines Objekts oder der Haut eines Körpers anzuordnen ist, einen Energiespeicher (220) und eine Kanüle (120), die aus einem Vorarbeitszustand, in dem die Kanüle (120) zurückgezogen ist, so dass sie nicht von der Hautkontaktoberfläche (140) vorsteht, in einen Arbeitszustand, indem die Kanüle (120) von der Hautkontaktoberfläche (140) vorsteht, unter Verwendung der Energie des Energiespeichers (220) bewegt werden kann, wobei die Bewegung von außerhalb der Kanülenanordnung (1) ausgelöst wird; **gekennzeichnet durch**
- eine wiederverwendbare Auslösevorrichtung (411-463), die mit der Kanülenanordnung (1) oder dem Energiespeicher (220) in der Weise verbunden werden kann, dass sie hiervon getrennt werden kann, um die Bewegung der Kanüle (120) auszulösen.

2. Kanülensystem nach Anspruch 1, wobei die Auslösevorrichtung (411-463) von der Kanülenanordnung (1) und/oder von dem Energiespeicher (220) getrennt ist.

3. Kanülensystem nach Anspruch 1 oder 2, wobei die Kanülenanordnung (1) ein Kanülenmodul (100), das die Hautkontaktoberfläche (140) und die Kanüle (120) aufweist, und ein Einsetzmodul (200), das den Energiespeicher (220) und einen Aktivierungsmechanismus (320, 370) aufweist, der von außerhalb der Kanülenanordnung (1) durch die Auslösevorrichtung (411-463) ausgelöst werden kann und dazu ausgelegt ist, zu verhindern, dass der Energiespeicher (220) entladen wird, bevor er durch die Auslösevorrichtung (411-463) ausgelöst wird, um so den Vorarbeitszustand der Kanüle (120) aufrecht zu erhalten, und dazu ausgelegt ist, den Energiespeicher (220) zur Entladung freizugeben, nachdem er durch die Auslösevorrichtung (411-463) ausgelöst worden ist, um so die Kanüle (120) in den Arbeitszustand zu drängen, umfasst.

4. Kanülensystem nach dem vorhergehenden Anspruch, wobei das Einsetzmodul (200) entworfen ist, um mit dem Kanülenmodul (100) lösbar gekoppelt zu werden.

5. Kanülenanordnung nach Anspruch 3 oder 4, wobei ein Blockierelement (320-365) vorgesehen ist, um zu verhindern, dass der Energiespeicher (220) entladen wird, das aus einer Blockierstellung freigegeben werden kann, um zu ermöglichen oder zu veranlassen, dass der Energiespeicher (220) entladen wird.

6. Kanülensystem nach Anspruch 5, wobei der Aktivierungsmechanismus (320-370) eine Auslöseschnittstelle (320-344) umfasst, die dazu ausgelegt ist, ein externes Auslösesignal zu empfangen und das Auslösesignal in eine Aktivierungswirkung umzusetzen, die auf das Blockierelement (320-365) oder auf ein Aktivierungselement (370) wirkt, um zu ermöglichen oder zu veranlassen, dass der Energiespeicher (220) entladen wird.

7. Kanülensystem nach Anspruch 6, wobei die Aktivierungswirkung eine mechanische und/oder magnetische und/oder elektrische und/oder elektromagnetische und/oder hydraulische und/oder thermische und/oder pneumatische und/oder pyrotechnische Aktivierungswirkung ist.

8. Kanülensystem nach einem der Ansprüche 3 bis 7, wobei der Aktivierungsmechanismus (320-370) einen Sicherheitsmechanismus (364) umfasst, der dazu ausgelegt ist, von einem Blockierzustand, in dem der Sicherheitsmechanismus (364) verhindert, dass der Aktivierungsmechanismus (320-370) ausgelöst wird oder der Blockiermechanismus (320-365) freigegeben wird, um zu verhindern, dass der Energiespeicher (220) entladen wird, in einen Freigabezustand zu schalten, indem der Energiespeicher (220) entladen werden kann oder in dem es möglich ist, auszulösen oder zu veranlassen, dass der Energiespeicher (220) entladen wird.

9. Kanülensystem nach einem der Ansprüche 5 bis 8 wobei:
der Aktivierungsmechanismus (320-370) ein Blockierelement (341-344) und ein Halteelement (350) umfasst;
das Blockierelement (341-344) an dem Halteelement (350) lösbar befestigt ist, um zu verhindern, dass der Energiespeicher (220) entladen wird;
das Blockierelement (341-344) dazu ausgelöst wird, von dem Halteelement (350) durch die Aktivierungswirkung gelöst zu werden, was veranlasst, dass der Energiespeicher (220) entladen wird; und
der Aktivierungsmechanismus (320-370) durch Bewegen des Blockierelements (341-344) ausgelöst werden kann.

10. Kanülensystem nach einem der Ansprüche 1 bis 9, wobei die Auslösevorrichtung (411-463) ein Teil einer medizinischen Vorrichtung oder einer Diabetes-Therapievorrichtung wie etwa eine Glukosedosiereinrichtung (450) oder eine Infusionspumpe oder ein Fluidverbinder (440) ist.

## Revendications

1. Système de canule, à utiliser avec une source de fluide ou une alimentation de liquide comme une pompe d'infusion pour délivrer un fluide ou un liquide à la canule (120), ledit système de canule comprenant :
- un ensemble de canule jetable (1) comprenant une surface (140) en contact avec la peau à placer sur une surface extérieure d'un objet ou sur la peau d'un corps, un accumulateur d'énergie (220) et une canule (120) qui peut être déplacée d'un état pré-opérationnel, dans lequel la canule (120) est rétractée de manière à ne pas faire saillie de la surface (140) en contact avec la peau , à un état opérationnel, dans lequel la canule (120) fait saillie de la surface (140) en contact avec la peau, en utilisant l'énergie de l'accumulateur d'énergie (220), dans lequel le déplacement est déclenché extérieurement à l'ensemble de canule (1) ; **caractérisé par**
- un dispositif de déclenchement réutilisable (411-463) qui peut être connecté, de manière à pouvoir être séparé, à l'ensemble de canule (1) ou à l'accumulateur d'énergie (220) afin de déclencher le déplacement de la canule (120).

2. Système de canule selon la revendication 1, dans lequel le dispositif de déclenchement (411-463) est distinct de l'ensemble de canule (1) et/ou de l'accumulateur d'énergie (220).

3. Système de canule selon la revendication 1 ou 2, dans lequel l'ensemble de canule (1) comprend un module de canule (100), comprenant la surface en contact avec la peau (140) et la canule (120), et un module d'insertion (200) comprenant l'accumulateur d'énergie (220) et un mécanisme d'activation (320, 370) qui peut être déclenché de l'extérieur de l'ensemble de canule (1) par le dispositif de déclenchement (411-463) et qui est adapté pour empêcher le déchargement de l'accumulateur d'énergie (220) avant son déclenchement par le dispositif de déclenchement (411-463), en maintenant ainsi l'état pré-opérationnel de la canule (120), et qui est adapté pour permettre à l'accumulateur d'énergie (220) d'être déchargé après son déclenchement par le dispositif de déclenchement (411-463), en forçant ainsi la canule (120) à être dans l'état opérationnel.

4. Système de canule selon la revendication précédente, dans lequel le module d'insertion (200) est conçu pour être couplé de manière libérable au module de canule (100).

5. Ensemble de canule selon la revendication 3 ou 4, dans lequel un élément de blocage (320-365) est prévu pour empêcher le déchargement de l'accumulateur d'énergie (220) et peut être libéré d'une position de blocage afin de permettre ou de causer un déchargement de l'accumulateur d'énergie (220).

6. Système de canule selon la revendication 5, dans lequel le mécanisme d'activation (320-370) comprend une interface de déclenchement (320-344) qui est apte à recevoir un signal de déclenchement externe et à convertir le signal de déclenchement en un effet d'activation qui agit sur l'élément de blocage (320-365) ou sur un élément d'activation (370) afin de permettre ou de causer le déchargement de l'accumulateur d'énergie (220).

7. Système de canule selon la revendication 6, dans lequel l'effet d'activation est un effet d'activation mécanique et/ou magnétique et/ou électrique et/ou électromagnétique et/ou hydraulique et/ou thermique et/ou pneumatique et/ou pyrotechnique.

8. Système de canule selon l'une quelconque des revendications 3 à 7, dans lequel le mécanisme d'activation (320-370) comprend un mécanisme de sécurité (364) qui est apte à passer d'un état de blocage dans lequel le mécanisme de sécurité (364) empêche le mécanisme d'activation (320-370) d'être déclenché ou le mécanisme de blocage (320-365) d'être libéré, ce qui empêche l'accumulateur d'énergie (220) d'être déchargé, à un état de libération dans lequel l'accumulateur d'énergie (220) peut être déchargé ou dans lequel il est possible de déclencher l'accumulateur d'énergie (220) ou de l'amener à être déchargé.

9. Système de canule selon l'une quelconque des revendications 5 à 8, dans lequel :
le mécanisme d'activation (320-370) comprend un élément de blocage (341-344) et un élément de rétention (350) ;
l'élément de blocage (341-344) est attaché de manière libérable à l'élément de rétention (350) afin d'empêcher le déchargement de l'accumulateur d'énergie (220) ; l'élément de blocage (341-344) est apte à être détaché de l'élément de rétention (350) par l'effet d'activation, ce qui amène l'accumulateur d'énergie (220) à être déchargé ; et
le mécanisme d'activation (320-370) peut être déclenché en déplaçant l'élément de blocage (341-344).

10. Système de canule selon l'une quelconque des revendications 1 à 9, dans lequel le dispositif de déclenchement (411-463) fait partie d'un dispositif médical ou d'un dispositif de thérapie de diabète, comme un glucomètre (450) ou un connecteur de fluide ou de pompe d'infusion (440).
